# EUROPEAN PATENT SPECIFICATION

(11) **EP 1 488 238 B1**
(45) Date of publication and mention of the grant of the patent: **18.11.2009**
(21) Application number: 03745056.6
(22) Date of filing: 21.03.2003
(51) Int. Cl.: G01N 33/564, G01N 33/68, G01N 33/573

(54) **SCREENING METHOD FOR GASTRITIS**
SCREENING-VERFAHREN FÜR GASTRITIS
METHODE DE DETECTION D'UNE GASTRITE

(30) Priority: 27.03.2002 SE 0200974
(43) Date of publication of application: 22.12.2004
(73) Proprietor: Atrophus AB, 582 25 Linköping (SE)
(72) Inventor: MARDH, Sven, S-590 77 Vreta Kloster (SE); MARDH, Erik, S-590 77 Vreta Kloster (SE)
(74) Representative: Inger, Lars Ulf Bosson
(86) International application number: PCT/SE2003/000469
(87) International publication number: WO 2003/081248

(56) References cited:
- WO-A1-00/67035
- LINDGREN ANDERS ET AL.: 'Serum antibodies to H+, K+-ATPase, serum pepsinogen A and helicobacter pylori in relation to gastric mucosa morphology in patients with low or low-normal concentrations of serum cobalamins' EUROPEAN JOURNAL OF GASTROENTEROLOGY & HEPATOLOGY vol. 10, 1998, pages 583 - 588, XP002966575
- ITO M. ET AL.: 'Role of anti-parietal cell antibody in helicobacter pylori-associated atrophic gastritis: evaluation in a country of high prevalence of atrophic gastrititis' SCANDINAVIAN JOURNAL OF GASTROENTEROLOGY vol. 37, 2002, pages 287 - 293, XP002966576
- MASANORI ITO ET AL.: 'Serological comparison of serum pepsinogen and antiparietal cell antibody levels between Japanese and German patients' EUROPEAN JOURNAL OF GASTROENTEROLOGY & HEPATOLOGY vol. 14, 2002, pages 123 - 127, XP002966577

## Description

### BACKGROUND OF THE INVENTION

Dyspepsia, or indigestion, is a common diagnosis in primary health care, but with poorly defined management. The annual prevalence of dyspepsia in the United Kingdom (UK) is about 25%, and in primary health care it accounts for 3-4%.of the consultations (Harris, A., Eur J Gastroenterol Hepat 1999; 11 (Suppl I): S31-5). Among the chronic disorders of the upper gastrointestinal tract are those which fall under the general category of gastritis. Gastritis is an inflammation of the stomach mucosa which is manifested by a broad range of poorly-defined symptoms such as indigestion, "heart burn" and excessive eructation. The typical means used to diagnose gastrointestinal disorders depends on such factors as the nature and severity of symptoms, the overall health of the individual, the medical history of the patient, the need for a specific diagnosis in order to implement a treatment with reasonable likellhood of success, and the availability of diagnostic devices.

Esophagogastroduodenoscopy (EGD) with histopathological examination of biopsies is the gold standard to determine the status of the gastric and duodenal mucosa. This examination is safe, accurate and sometimes indispensable, e.g., in the older age group and especially in the presence of alarm symptoms such as weight loss, anorexia, dysphagia, or gastrointestinal blood loss. The demand for upper endoscopy is increasing and in the UK approximately 0.5% of the population undergo this examination each year (Working Party of the Clinical Services Committee of the British Society of Gastroenterology, Provision of gastrointestinal endoscopy and related services for a district general hospital. Gut 1991; 32: 95-105; Gear, M.W.L., and Wilkinson, S.P., Br J Hosp Med 1989; 41: 438-44). Without EGD and visual inspection of the mucosa, gastritis is difficult to diagnose. However, EGD is expensive, inconvenient for the patient, and generally not recommended for children or patients with severe cardiopulmonary disease. Thus, for patients not having severe symptoms, a precise diagnosis of a gastrointestinal disorder might not be attempted. Such patients may simply be treated with conventional therapies, such as with antacids or drugs which inhibit stomach acid secretion. While such therapies might provide temporary relief of the symptoms, a cure is not often achieved. More effective treatments generally depend on a better diagnosis of the actual underlying gastrointestinal disorder. For example, many gastrointestinal disorders are mediated by bacterial infection of the mucosa, in which case treatment of the bacterial infection would most likely be required to effectively treat the manifested gastrointestinal disorder.

There is a need for a simple pre-gastroscopic screening method to reduce the endoscopy workload, and attempts have been made in this direction with some success in patients with uncomplicated simple dyspepsia (Bodger, K., et al., Scand J Gastroenterol 1999; 34: 856-63; and Moayyedi, R, et al., Eur J Gastroenterol Hepatol 1999; 11:1245-50). In young dyspeptic patients (< 40 years) screening for Helicobacter pylori (H. pylori) infection and a treatment strategy based on the presence of an infection reduces the endoscopy workload. This strategy appears as effective as an endoscopy-based strategy in reducing dyspeptic symptoms, dyspepsia consultation rates and the prescription of anti-secretory drugs (Moayyedi, P., et al., Eur J Gastroenterol Hepatol 1999; 11:1245-50). In the elderly with dyspepsia, however, the prevalence of gastritis and its consequences are considerably higher pointing at EGID as the initial diagnostic step.

The inflamed gastric mucosa transmits specific information to the blood stream that allows diagnosis of gastritis by serologic analysis. The determination of antibodies to H⁻K⁺-ATPase, concentration of serum pepsinogen A and antibodies to *H. pylori* for diagnosing gastric body mucosal atrophy was shown (Lindgren, A., et al., Eur J Gastroenterol Hepathol 1998;10:583-88). The morphology and cellular composition of the mucosa vary between the acid secreting corpus and the antrum. This may aid to distinguish corpus and pangastritis from antral gastritis. A number of serological markers have been described. Infection with H. pylori is the major cause of chronic gastritis, duodenal ulcer, mucosa associated lymphoid tissue (MALT) lymphoma and gastric cancer (Chiba, N., et al., Can Fam Physician 1998.; 44: 1481 -8; Genta, R.M., Gut 1998; 43: 35-8; Coyle, W.J., et al., Gastrointest Endosc 1998; 48: 327-8; Lee, B.M., et al., Jpn J Cancer Res 1998; 89: 597-603), and antibodies to various H. pylori antigens can easily be detected in the blood (Bodger, K, et al., Scand J Gastroenterol 1999; 34: 856-63; Moayyedi, P., et al., Eur J Gastroenterol Hepatol 1999; 11:1245-50). This infection is sometimes associated with an autoimmune reaction leading to atrophy of the corpus mucosa (Ozasa, K, et al., Dig Dis Sci 1999; 44: 253-6). A common feature of gastric autoimmunity and frequently several other autoimmune diseases, e.g., thyroiditis, insulin dependent diabetes mellitus and sometimes rheumatoid arthritis, is the occurrence of parietal cell autoantibodies (Bech, K, et al., Acta Endocrinol.1 991, 124: 534-9; Barrio, R., et al., Pediatr Endocrinol Metab 1997, 10: 511-6; Datta, A., et al., Indian J Med Res 1990, 92: 228-32; Mårdh, S., et al., Scand J Gastroenterol 1991, 26: 1089-96). The parietal cell H,K-ATPase a- and R-subunits were found to be the major autoantigens in autoimmune atrophic gastritis (Karisson, A., et al., J Clin Invest 1988, 81A75-9; Song, Y.H., et al., Scand J Gastroenterol 1994, 29:122-7; Ma, J.Y., et al., Scand J Gastroenterol 1994, 20: 790-4). A low titre of H,K-ATPase antibodies is normally found in healthy individuals due to the normal turn-over of parietal cells. In patients with inflamed corpus mucosa the titre may be increased.

Pepsinogen 1 (PGI) is secreted by the chief and mucous neck cells of the corpus mucosa into the lumen of the stomach but a small fraction (about 1 %) leaks into the blood stream (Baron, JR, Clinical tests of gastric secretion: History,' Methodology and Interpretation. (1978) London: Macmillan). Increased serum concentrations of PG1 are frequently found in patients with duodena) ulcer (Samloff, I.M., et al., Gastroenterol 1975 Jul, 69(1). 83-90). In patients with pernicious anaemia due to severe atrophy of the corpus mucosa serum PG1 is significantly reduced (Samloff, I.M., et al., Gastroenterol 1982 Jul, 83(1 Pt 2): 204-9).

Existing, non-invasive methods of detecting gastrointestinal disorders include monitoring blood flow to the affected region to detect inflammation (US 5;524,622). A significant disadvantage of this method is the requirement of injecting multiple substances into the patient followed by the detection by gamma camera. Additionally, the method only detects inflammation, and does not address the underlying cause of any inflammation. Other methods of detecting gastrointestinal disorders include assays for individual analytes such as pepsinogen (US 5,879,897) or Helicobacter pylori (US 5,814,455; 6,067,989; 6,068,985; 6,090,611). Other serological markers are gastrin (Borch, K, et al., Scand J Gastroenterol 1997, 32:198-202), pepsinogen 11 (Carmel, R., Am J Pathol 1998, 90: 442-5), intrinsic factor antibodies (Waters, H.M., et al., J Clin Pathol 1989, 42: 307-12) and pepsinogen antibodies (Mårdh, S., et al., Acta Physiol Scand 1989, 136: 581-7). Although each one of these markers may be used to diagnose changes in the gastric mucosa, the overlap between healthy subjects and patients is great, and even greater among the various subgroups of patients. Therefore none of all these markers alone is sufficient for a reliable diagnosis.

### SUMMARY OF THE INVENTION

The instant invention provides a screening method for gastritis in its various forms involving the evaluation of assay results for H,K-ATPase antibodies, H. pylori antibodies, and serum pepsinogen 1 concentration. The analysis of multiple analytes associated with gastritis provides a reliable indication of various subgroups of gastritis.

The invention describes a method for diagnosing possible presence of gastritis in a human by evaluating a blood sample, comprising assaying the blood sample for the presence of antibodies specific for H,K-ATPase, antibodies specific for Helicobacter pylori, and the concentration of pepsinogen I, whereby the presence of H,K-ATPase antibodies, Helicobacter pylori antibodies, and pepsinogen I concentration are compared between themselves and in relation to the respective values of H,K-ATPase antibodies Helicobacter pylori antibodies, and pepsinogen concentration of a normal population and an additional indicator consisting of level of pepsinogen I multiplied by the level of Helicobacter pylori antibodies, and wherein the level of this additional indicator is compared to a standard, in a software related system, wherein altered levels in the sample is indicative of gastritis, and whereby, preferably, an altered level detection leads to the issuance of a remittance for further investigation with regard to gastritis.

A preferred embodiment relates to a method, wherein the step of determining the levels of said indicators comprises performing immunoassays for detecting the indicators.

A another preferred embodiment relates to a method, wherein the group of indicators and includes an additional indicator consisting of the level of pepsinogen I multiplied by the level of Helicobacter pylori antibodies, and wherein the level of this additional indicator is compared to a standard.

A further other preferred embodiment relates to a method, wherein the levels of H,K-ATPase antibodies and Helicobacter pylori antibodies, which are significantly higher than the levels in a normal population are indicative of gastritis.

A further preferred embodiment relates to a method, wherein a lowered level of pepsinogen I concentration is indicative of corpus atrophy.

A preferred other embodiment relates to a method, wherein an increased level of pepsinogen I concentration is indicative of a corpus gastritis, optionally without any autoimmunity Involved.

A further other preferred embodiment relates to a method, wherein the level of H,K-ATPase antibodies differing from that of the normal population is indicative of an autoimmune corpus atrophy.

A another preferred embodiment relates to a method, wherein the level of Helicobacter pylori antibodies differing from that of the normal population is indicative of antrum, or pangastritis.

A further preferred embodiment relates to a method, wherein increased levels of Helicobacter pylori antibodies, and normal to lowered concentrations of pepsinogen I are indicative of atrophy.

A preferred other embodiment relates to a method, wherein very low concentrations of pepsinogen I in combination with increased levels of H,K-ATPase antibodies are indicative of corpus atrophy.

### BRIEF DESCRIPTION OF THE DRAWINGS

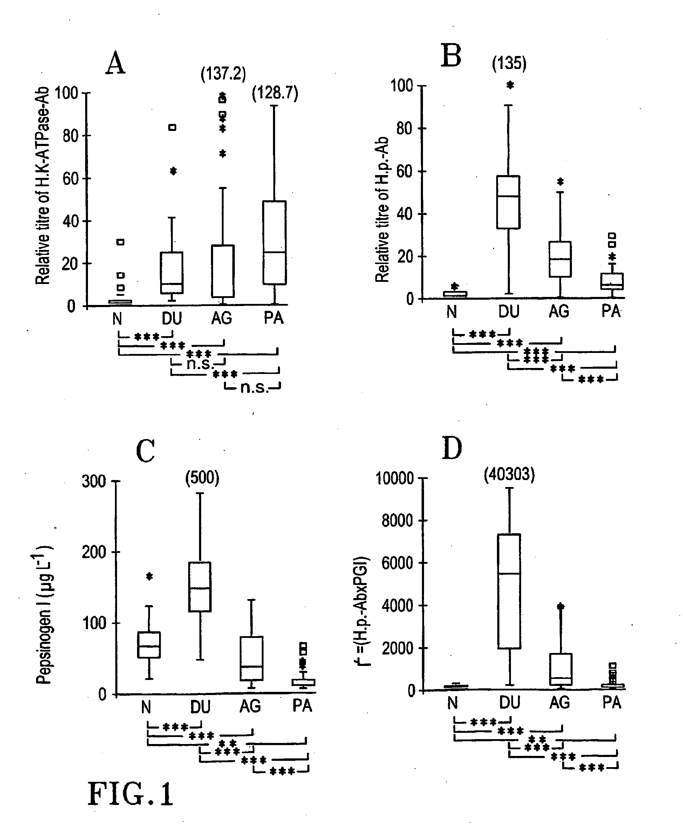

FIGS. 1 A-1 D are graphs showing the levels of H,K-ATPase antibodies, H. pylori antibodies, pepsinogen 1, and result of H. pylori antibody level multiplied by pepsinogen 1 concentration, respectively, for individuals with normal gastric mucosa (N) and those with duodenal ulcer (DU), atrophic gastritis (AG) and gastritis with pernicious anaemia (PA).

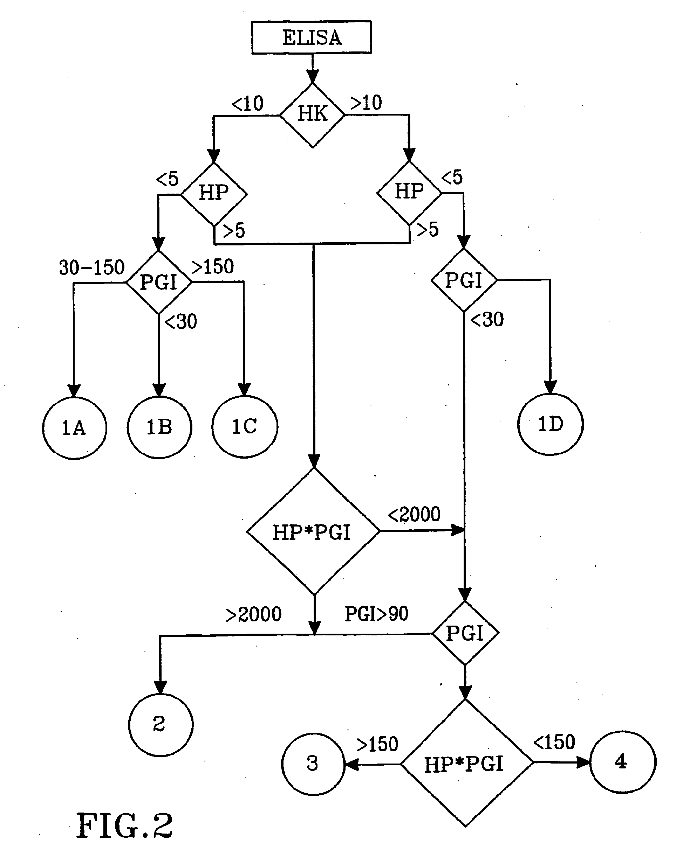

FIG. 2 is a flow chart depicting a testing evaluation scheme.

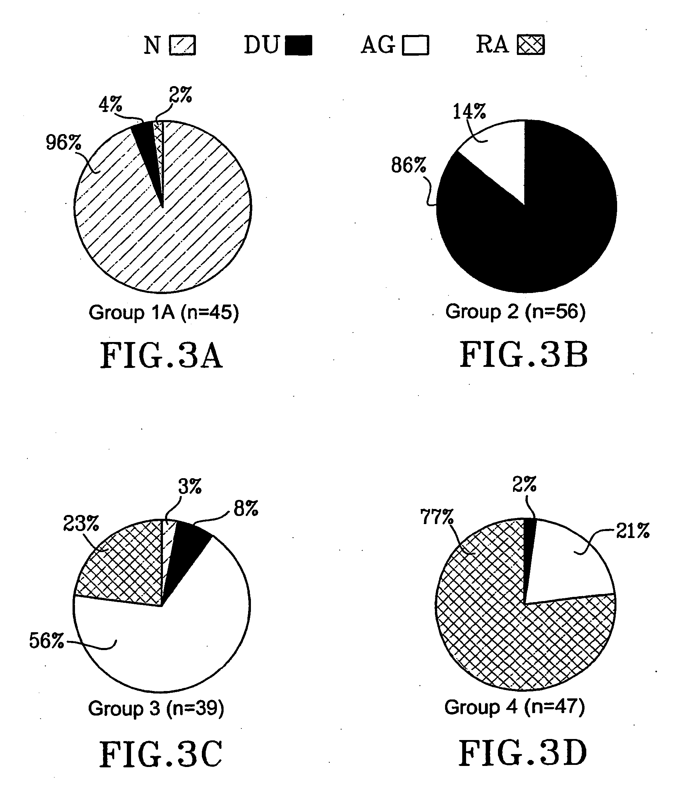

FIGS. 3A-3D are pie charts showing the four major groups identified by the screening tests of the instant invention for the test groups N, DU, AG, and PA.

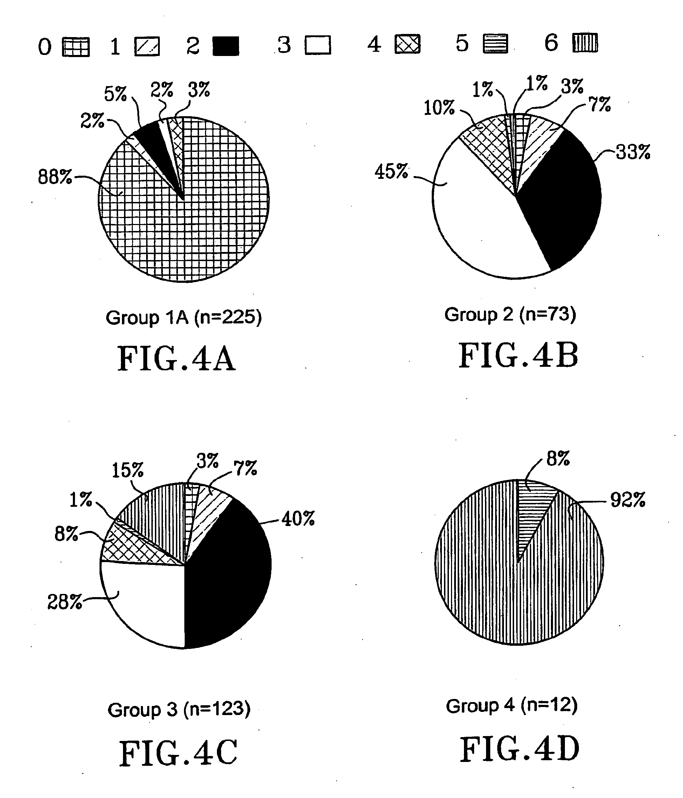

FIGS. 4A-4D are pie charts showing the four major groups identified by the screening tests of the instant invention for the general population.

### DETAILED DESCRIPTION OF THE INVENTION

Gastritis and dyspepsia are common entitles in primary health care but with poorly defined management. The aim of the present invention is to provide a serologic screening test for gastritis. Esophagogastroduodenoscopy (EGD) with biopsy and histological examination requires a skilful and experienced staff and it is presently the only reliable technique for diagnosing gastritis, benign ulcer and neoplasia. The last two groups are generally closely associated with chronic gastritis. Previous investigations often concluded that serology has a limited diagnostic value. However, the instant evaluation scheme for analyzing serology data is useful as a pre-gastroscopic screening of dyspepsia, irrespective of the age of the patient, which is a remarkable improvement compared with previous reports (Bodger, K, et al., Scand J Gastroenterol 1999, 34: 856-63; Moayyedi, R, et al., Eur J Gastroenterol Hepatol 1999, 11:1245-50). The immune system and chemical signalling from the inflamed gastric mucosa provide serum analytes and diagnostic possibilities for detection of gastritis.

Assay methods for determining the level of H,K-ATPase antibodies, Helicobacter pylori antibodies and pepsinogen 1 concentration are known. In a preferred embodiment of the instant invention, the method of determining the level of the analytes is by immunoassay. The immunoassay may be any of the well-known methods, including, but not limited to, enzyme-linked-immunosorbent assays (ELISA), enzyme immunoassay (EIA), radioimmunoassay (RIA), immunoprecipitation (IP), and optical or electrochemical detection of immunoligand interaction. In a preferred embodiment, the immunoassay is one in which the antigen is immobilized on a solid support, samples added, followed by labelled antibody.

The immunoassay methods are based on analyses of a blood sample (or plasma or serum) from patients; the autoantibodies against H,K-ATPase, antibodies against Helicobacter pylori and concentration of pepsinogen are assayed. All of these analytes serve as markers of an inflammatory condition in the gastric mucosa. The methods of detecting the analytes, such as immunoassays, are well-known in the art.

The assay results are analysed by a new grouping procedure in which the results are compared with reference values from healthy individuals from the normal population. A mathematical expression (the product of pepsinogen concentration multiplied by the titre of Helicobacter pylori antibodies) is essential to identify one particular group of patients. Other mathematical procedures may be used provided they achieve determination of a useful grouping of gastritis patients. This grouping procedure diagnoses inflammatory conditions in the gastric mucosa that previously have required the more costly and complicated gastroscopy with histopathological examination of biopsies from the mucosa.

The reference values from healthy individuals from the normal population may be standardized for each assay. In this embodiment, the test results are compared to standardized reference values in order to determine the patient's likelihood of having gastritis. In an alternative embodiment, the reference values are obtained by testing a normal control along with the patient samples. When performing the assays, one would run the assay on the control and standards at the same time the patient samples are tested. The assay results for the control and standards would then be compared to the results for the patient samples.

Sera from subjects examined with gastroscopy and biopsy were analysed for H,K-ATPase antibodies, Helicobacter pylori antibodies, and pepsinogen 1. The diagnoses were normal gastric mucosa (n=50), duodenal ulcer (n=53), and atrophic corpus gastritis with (n=50) or without pernicious anaemia (n=46). An evaluation scheme (flow chart) was constructed to optimise the diagnostic agreement between serology and gastric mucosal morphology. Four major serologic groups and thirteen subgroups were obtained with an over-all sensitivity to detect gastritis of 98% (146/149) (95% Cl 94-100%) and a specificity of 84% (42/50) (95% Cl 71-93%). Additional sera from 483 subjects from the general population were grouped by serology. The overall sensitivity to detect gastritis in this population was 88% (211/240) (95% Cl 83-92%) with a specificity of 81% (196/243) (95% Cl 75-85%), There was a good, agreement between serology and the gastric mucosal morphology both in the groups used for developing the evaluation scheme and in the sample of the general population. Thus, serology is appropriate for initial identification of subjects with a normal gastric mucosa, those who qualify for eradication of Helicobacter pylori, and those who are at risk of developing malignancy and therefore require gastroscopic examination.

In reading the assay results, altered levels of the analytes in a patient sample as compared to normal control values is indicative of the patient having gastritis. By "altered" is meant levels either significantly above or significantly below the levels of the normal control. What is significant depends on the accuracy and precision of the specific test performed and may be determined empirically without undue experimentation. Additionally, the levels of multiple analytes in a patient sample are compared to normal control values in order to obtain a more accurate determination of whether or not the patient has gastritis. For example, in general, patient levels of H,K-ATPase antibodies and Helicobacter pylori antibodies above normal control levels is indicative of gastritis, while patient levels of the pepsinogen concentration either significantly above (duodenal ulcer) or below (severe atrophy of the corpus mucosa) normal control levels may be indicative of gastritis. The pepsinogen concentration multiplied by the Helicobacter pylori antibody titre either significantly above or significantly below the normal control may be indicative of gastritis. In order to determine the likelihood of the patient having gastritis and/or to determine the subgroup of the patient's gastritis, the combination of multiple assay results are compared to normal control values. Because some analytes may be either higher or lower than normal control levels and still be indicative of gastritis, the comparison of levels of multiple analytes in a patient sample to normal control levels of the same analytes provides a more accurate determination of gastritis.

With histomorphological examination as the gold standard, the present invention demonstrates that a combination of serologic assays detects 87% (196/225, group 1A in FIG, 2) of subjects with normal gastric mucosa in a sample of the general population and in the study groups it was 84% (42/50, group 1A). In the study groups, serology detected 91 % (48/53, group 2) of subjects with duodenal ulcer and 84% (81±96, groups 3 plus 4) of subjects with corpus predominant atrophic gastritis with or without pernicious anaemia. All subjects with pernicious anaemia were obtained in group 4. In the population sample, groups 2A-D comprised 15%. Among these, only 3% had a normal mucosa, while antral gastritis with atrophy and pangastritis overall comprised 88%. All subjects in groups 2A-D were H. pylori-positive. It is therefore suggested that subjects aged 40-50 years or less (Bodger, K, et al., Scand J Gastroenterol 1999, 34: 856-63; Moayyedi, R, et al., Eur J Gastroenterol Hepatol 1999, 11 *1245-50) that are obtained in groups 2A-D may be treated according to the current recommendations for peptic ulcer disease.

Only 12 subjects in the population sample were obtained in group 4A4C; one had non-atrophic corpus predominant gastritis and the remaining 11 atrophic corpus predominant gastritis. Thus, subjects belonging to group 4 should be recommended EGID due to the increased risk of malignancy.

In the population sample there were 12% (29/225) with normal gastric mucosa, but abnormal results of the serological analysis. There are probably several explanations for this discrepancy; some results of serum analytes may reflect a previous inflammatory condition, or the ELISAs may be more sensitive than the most experienced examiner to detect small changes in the mucosa.

In conclusion, EGID with biopsy remains the gold standard for an accurate diagnosis of the status of the gastric mucosa. However, as the following examples show, serology is a good complement when combined with the patient's symptomatology and medical history. It functions as a "serologic biopsy". Therefore, serologic assays are well-suited for pre-gastroscopic screening of dyspepsia to identify: (a) patients with normal gastric mucosa (group 1A), (b) H. pylori-positive patients with high levels of serum PG1 and high "H.p. x PG1 -factor" (group 2), and (c) patients with corpus predominant atrophic gastritis (groups 3 plus 4). Patients in (a) may be examined further for "non-acid-related" disorders, those in (b) younger than 40-50 years may be treated according to the recommendations for peptic ulcer disease, and in cases with unsuccessful treatment, the patients should be referred to EGD. Patients in (c) are at a higher risk of developing gastric malignancy and should therefore be referred to EGD.

The described serologic assays and the evaluation procedure are simple and may be performed in any clinical laboratory with some experience in immunoassays. They provide a reduction in the endoscopic workload, are beneficial for the patient, provide a valuable diagnostic too) for the doctor and are cost-efficient.

### EXAMPLE 1

For the initial evaluation of the serologic results, sera from four groups of subjects examined endoscopically and histologically were selected; 50 subjects with normal gastric mucosa (N) (38 males and 12 females, median age 63 years, range 37-80), 53 subjects with acute duodenal ulcer (DU) (39 males and 14 females, median age 52 years, range 20-79), 46 subjects diagnosed as having mild to severe corpus , predominant atrophic gastritis (AG) (23 males and 23 females, median age 68 years, range 40-82), and 50 subjects with corpus predominant atrophic gastritis with pernicious anaemia (PA) (23 males and 27 females, median age 68 years, range 40-83). The criteria for the diagnosis of pernicious anaemia which included a Schilling test showing intrinsic factor deficiency have previously been given (Borch, K, et al., Scand J Gastroenterol 1984, 19: 154-60).

### EXAMPLE 2

A sample of 483 subjects (266 males and 217 females, median age 65, range 37 to 85 years) randomly selected from a general population in Sweden was examined with EW with biopsy and blood sampling. Results of this study have recently been published (Borch, K, et al., Dig Dis Sci, 2000, 45: 1322-29). In biopsy specimens, gastritis was classified according to the Sydney system into antrum predominant-, corpus predominant- and pangastritis with or without atrophy and with or without presence of H. pylori (Price, A., J Gastroenterol Hepatol 1991, 6: 209-22; Dixon, M.F., et al., Am J Surg Pathol 1996, 20: 1161-81). EW was performed as previously described (Borch,K, et al., Dig Dis Sci, 2000, 45: 1322-29). Three biopsies were taken from the gastric body (major-, anterior-, and posterior aspect), and the antrum within 3 cm of the pylorus.

### EXAMPLE 3

### Analysis of serum pepsinogen 1 (PG1)

The assay was based on a non-competitive sandwich technique using a horseradish peroxidase (HRP)-labelled antibody specific for PG1 to detect PG1 bound to a stationary antibody. The latter antibody was immobilized on a microtitre plate and had affinity for a different antigenic site in PG1 than the enzyme-labelled antibody.

Assay of serum pepsinogen 1 (PG1) was carried out using Gastroset PG1 (Gastroset PG1 Cat. No. 67882, Orion Diagnostica, Espoo, Finland) according to the manufacturer's instructions. Aliquots of 20 µL of standards, control and serum sample were added in duplicate into microtitre wells precoated with stationary pepsinogen 1 antibody. Assay buffer (100 µL) was added and the microtitre wells incubated for 30 minutes, washed twice, and then incubated for another 30 minutes with horseradish peroxidase-labelled PGI (HRP-PGI) antibody diluted 1:100 with assay buffer. The wells were washed four times and then incubated with substrate solution for 30 minutes; the reaction was stopped and colour development measured in an ELISA-reader.

### Statistical Analysis

Results are presented as median and interquartile range (25th-75th percentiles). Proportions (percentages) are given with 95 percent confidence interval (Cl), when considered relevant. Wilcoxon's signed-rank test was used to evaluate differences between pairs of patient groups. The level of significance was p<0.05.

### References

1. Harris A, Dyspepsia and Helicobacter pylori: test, treat or investigate? Eur J Gastroenterol Hepat 1999; 11 (Suppi I): S31-5.
2. Working Party of the Clinical Services Committee of the British Society of Gastroenterology. Provision of gastrointestinal endoscopy and related services for a district general hospital. Gut 1991, 32: 95-105.
3. Gear MWL, Wilkinson SP, Open access upper alimentary endoscopy. Br J Hosp Med 1989; 41: 438-44.
4. Bodger K, Wyatt J.1, Heatley RV, Serologic screening before endoscopy: The value of Helicobacter pylori serology, serum recognition of the CagA and VacA proteins, and serum pepsinogen 1, Scand J Gastroenterol 1999; 34: 856-63.
5. Moayyedi P, Zilles A, Clough M, Hemingbrough E, Chalmers DIVI, Axon AT, The effectiveness of screening and treating HeUcobacter pylori in the management of dyspepsia, Eur J Gastroenterol Hepatol 1999; 11: 1245-50.
6. Chiba N, Lahaie R, Fedorak RN, Bailey R, Veldhuyzen van Zanten SJ, Bernucci B, Helicobacter pylori and peptic ulcer disease. Current evidence for management strategies, Can Fam Physician 1998, 44: 1481-8.
7. Genta RM, Acid suppression and gastric atrophy: sifting fact from fiction, Gut 1998; 43: 35-8.
8. Coyle WJ, Lawson JM, Helicobacter pylori infection in patients with early gastric cancer by the endoscopic phenol red test, Gastrointest Endosc 1998; 48: 327-8.
9. Lee BM, Jang JJ, Kim JS, You YC, Chun SA, Kim HS, et al., Association of Helicobacter pylori infection with gastric adenocarcinoma, Jpn J Cancer Res 1998; 89: 597-603.
10. Ozasa K, Kurata JH, Higashi A, K. Hayashi K, Inokuchi H, Miki K, et al., Helicobacter pylori infection and atrophic gastritis: a nested case-control study in a rural town in Japan, Dig Dis Sci 1999; 44: 253-6.
11. Bech K, Hoier-Madsen M, Feldt-Rasmussen U, Jensen BM, Molsted-Pedersen L, KuN C, Thyroid function and autoimmune manifestations in. insulin -dependent diabetes mellitus during and after pregnancy, Acta Endocrinol 1991; 12k534-9.
12. Barrio R, Roldan MB, Alonso M, Canton R, Camarero C, Helicobacter pylori infection with parietal cell antibodies in children and adolescents with insulin dependent diabetes meflitus, Pediatr Endocrinol Metab 1997; 10: 511-6.
13. Datta A, Deodhar SI), Datta U, Sehgal S, Non-organ specific & organ specific antibodies in rheumatoid arthritis, Indian J Med Res 1990; 92: 228-32.
14. MArdh S, Ma JY, Song YH, Aly A, Henriksson K, Occurrence of autoantibodies against intrinsic factor, H,K-ATPase and pepsinogen in atrophic gastritis and rheumatoid arthritis. Scand J Gastroenterol 1991; 26: 1089-96.
15. Karisson A, Burman P, L66f L, MArdh S, Major parietal cell antigen in autoimmune gastritis with pernicious anernia is the acid-producing H,K-adenosine triphosphatase of the stomach, J Clin Invest 1988; 81: 475-9.
16. Song YH, Ma JY, MArdh S, Liu T, Sjbstrand SE, Rask L et al., Localization of a pernicious anernia-autoanti.body epitope on the a-subunit of the human H,K-ATPase. Scand J Gastroenterol 1994; 29: 122-7.
17. Ma JY, Borch K, IVIkdh S, Human gastric H,K-adenosine triphosphatase R-subunit is a major autoantigen in atrophic corpus gastritis. Expression of the recombinant human glycoprotein in insect cells. Scand J Gastroanterol 1994; 20: 790-4.
18. Baron JH, Clinical tests of gastric secretion: History, Methodology and Interpretation. (1978) London: Macmillan.
19. Sarnloff iM, Liebman WM, Panitch NIVI, Serum group 1 pepsinogens by radioimmunoassay in control subjects and patients with peptic ulcer. Gastroenterol 1975 Jul; 69(I): 83-90.
20. Samloff IM, Varis K, Ihamaki T, Siurala M, Rotter J11, Relationships among serum pepsinogen 1, serum pepsinogen 11, and gastric mucosal histology. A study in relatives of patients with pernicious anemia. Gastroenterol 1982 Jul; 83(1 Pt 2): 204-9.
21. Borch K, Stridsberg M, Burman P, Rehfeld J17, Basal chromogranin A and gastrin concentrations in circulation correlate to endocrine cell proliferation in type A gastritis. Scand J Gastroenterol 1997; 32:198-202.
22. Carmel R, Pepsinogens and other serum markers in pernicious anemia. Am J Pathol 1998; 90: 442-5.
23. Waters HM, Smith C, Howarth JE, Dawson DW, Delarnore IW, New enzyme immunoassay for detecting total, type 1, and type 11 intrinsic factor antibodies. J Clin Pathol 1989; 42: 307-12.
24. MArdh S, Song Y-H, Characterization of antigenic structures in autoimmune atrophic gastritis with pernicious anemia. The parietal cell H,K-ATPase and the chief cell pepsinogen are the two major antigens. Acta Physiol Scand 1989; 136: 581-7.
25. Borch K, Liedberg G, Prevalence and incidence of pernicious anemia*. An evaluation for gastric screening. Scand J Gastroenterol 1984; 19: 154-60.
26. Borch K, Mnsson K-A, Rede'en S, Petersson F, MArdh S, Franz6n L, Prevalence of gastroduodenitis and Helicobacter pylori infection in the general population: Relations to symptomatology and life style. Dlg Dis Sci, 2000, 45: 1322-29.
27. Price A, The Sydney system: Histological division, J Gastroenterol Hepatol 1991; 6: 209-22.
28. Dixon MF, Genta RM, Yardley JH, Correa P, the Participants in the International Workshop on the Histopathology of Gastritis, Houston 1994. Classification and grading of gastritis. The updated Sydney system, Am J Surg Pathol 1996; 20: 1161-81.
29. Karisson FA, Burman R 1-65f L, Oisson M, Scheynius A, MArdh S, Enzyme-linked immunosorbent assay of H,K-ATPase, the parietal cell antigen. Clin exp Immunol 1987; 70:604-10.
30. Ma J-Y, Borch K, Sj6strand SE, Janzon L, MArdh S, Positive correlation between H,K-adenosine triphosphatase autoantibodies and Helicobacter pylori antibodies in patients with pernicious anemia. Scand Gastroenterol 1994; 29: 961-6.

## Claims

1. A method for diagnosing possible presence of gastritis in a human by evaluating a blood sample, comprising assaying the blood sample for the presence of antibodies specific for H,K-ATPase, antibodies specific for Helicobacter pylori, and the concentration of pepsinogen I, whereby the presence of H,K-ATPase antibodies, Helicobacter pylori antibodies, and pepsinogen I concentration as well as an additional indicator consisting of the level of pepsinogen I multiplied by the level of Helicobacter pylori antibodies, are compared between themselves and in relation to the respective values of H,K-ATPase antibodies, Helicobacter pylori antibodies, and pepsinogen I concentration of a normal population, and wherein the level of this additional indicator is compared to a standard, in a software related system,
wherein altered levels in the sample is indicative of gastritis.

2. A method according to claim 1, wherein the step of determining the levels of said indicators comprising performing immunoassays for detecting the indicators.

3. A method according to one or more of claims 1-2, wherein the levels of H,K-ATPase antibodies and Helicobacter pylori antibodies, which are significantly higher than the levels in a normal population is indicative of gastritis.

4. A method according to one or more of claims 1-2, wherein a lowered level of pepsinogen I concentration is indicative of corpus atrophy.

5. A method according to one or more of claims 1-2, wherein an increased level of pepsinogen I concentration is indicative of a corpus gastritis.

6. A method according to one or more of claims 1-2, wherein an increased level of pepsinogen I concentration is indicative of a corpus gastritis, without any autoimmunity involved.

7. A method according to one or more of claims 1-2, wherein the level of H,K-ATPase antibodies differing from that of the normal population is indicative of an autoimmune corpus atrophy.

8. A method according to one or more of claims 1-2, wherein the level of Helicobacter pylori antibodies differing from that of the normal population is indicative of antrum, or pangastritis.

9. A method according to one or more of claims 1-2, wherein increased levels of Helicobacter pylori antibodies, and normal to lowered concentrations of pepsinogen I is indicative of atrophy.

10. A method according to one or more of claims 1-2, wherein very low concentrations of pepsinogen I in combination with increased levels of H,K-ATPase antibodies is indicative of corpus atrophy.

## Patentansprüche

1. Verfahren zur Diagnose eines möglichen Vorliegens von Gastritis in einem Menschen durch Beurteilung einer Blutprobe, umfassend das Untersuchen der Blutprobe auf das Vorliegen von für H,K-ATPase spezifischen Antikörpern, von für Helicobacter pylori spezifischen Antikörpern und auf die Konzentration von Pepsinogen I, wobei das Vorliegen von H,K-ATPase-Antikörpern, Helicobacter pylori-Antikörpern und die Pepsinogen I-Konzentration sowie ein zusätzlicher Indikator, bestehend aus dem Spiegel von Pepsinogen I, multipliziert mit dem Spiegel von Helicobacter pylori-Antikörpern, untereinander und in bezug auf die jeweiligen Werte von H,K-ATPase-Antikörpern, Helicobacter pylori-Antikörpern und Pepsinogen I-Konzentration einer normalen Population verglichen werden, und wobei der Spiegel dieses zusätzlichen Indikators mit einem Standard verglichen wird, in einem Software-bezogenen System, worin veränderte Spiegel in der Probe für Gastritis indikativ sind.

2. Verfahren gemäss Anspruch 1, worin der Schritt der Bestimmung der Spiegel der Indikatoren das Durchführen von Immuntests zum Nachweis der Indikatoren umfasst.

3. Verfahren gemäss einem oder mehreren der Ansprüche 1 bis 2, worin die Spiegel der H,K-ATPase-Antikörper und Helicobacter pylori-Antikörper, die signifikant höher sind als die Spiegel in einer normalen Population, für Gastritis indikativ sind.

4. Verfahren gemäss einem oder mehreren der Ansprüche 1 bis 2, worin ein erniedrigter Spiegel der Pepsinogen I-Konzentration für eine Korpusatrophie indikativ ist.

5. Verfahren gemäss einem oder mehreren der Ansprüche 1 bis 2, worin ein erhöhter Spiegel der Pepsinogen I-Konzentration für eine Korpusgastritis indikativ ist.

6. Verfahren gemäss einem oder mehreren der Ansprüche 1 bis 2, worin ein erhöhter Spiegel der Pepsinogen I-Konzentration für eine Korpusgastritis indikativ ist, ohne dass eine Autoimmunität beteiligt ist.

7. Verfahren gemäss einem oder mehreren der Ansprüche 1 bis 2, worin der Spiegel der H,K-ATPase-Antikörper, der sich von dem der normalen Population unterscheidet, für eine autoimmune Korpusatrophie indikativ ist.

8. Verfahren gemäss einem oder mehreren der Ansprüche 1 bis 2, worin der Spiegel der Helicobacter pylori-Antikörper, der sich von dem der normalen Population unterscheidet, für eine Antrum- oder Pangastritis indikativ ist.

9. Verfahren gemäss einem oder mehreren der Ansprüche 1 bis 2, worin erhöhte Spiegel der Helicobacter pylori-Antikörper und normale bis erniedrigte Konzentrationen an Pepsinogen I für eine Atrophie indikativ sind.

10. Verfahren gemäss einem oder mehreren der Ansprüche 1 bis 2, worin sehr niedrige Konzentrationen an Pepsinogen I zusammen mit erhöhten Spiegeln der H,K-ATPase-Antikörper für eine Korpusatrophie indikativ sind.

## Revendications

1. Procédé pour diagnostiquer la présence éventuelle d'une gastrite chez un être humain en évaluant un échantillon de sang, comprenant le dosage dans l'échantillon de sang, de la présence d'anticorps spécifiques pour la H,K-ATPase, d'anticorps spécifiques pour Helicobacter pylori et de la concentration du pepsinogène I, dans lequel la présence d'anticorps de la H,K-ATPase, d'anticorps de Helicobacter pylori et de la concentration de pepsinogène I ainsi que d'un indicateur supplémentaire consistant en le niveau de pepsinogène I multiplié par le niveau d'anticorps de Helicobacter pylori, sont comparés entre eux et en relation avec les valeurs respectives d'anticorps de la H,K-ATPase, d'anticorps de Helicobacter pylori et de concentration de pepsinogène I d'une population normale, et dans lequel le niveau de cet indicateur supplémentaire est comparé à une norme, dans un système associé à un logiciel, où les niveaux modifiés dans l'échantillon sont indicatifs d'une gastrite.

2. Procédé selon la revendication 1, dans lequel l'étape de détermination des niveaux desdits indicateurs comprend la réalisation d'essais immunologiques pour détecter les indicateurs.

3. Procédé selon une ou plusieurs des revendications 1 - 2, dans lequel les niveaux d'anticorps de la H,K-ATPase et d'anticorps de Helicobacter pylori, qui sont significativement supérieurs aux niveaux observés dans une population normale, sont indicatifs d'une gastrite.

4. Procédé selon une ou plusieurs des revendications 1-2, dans lequel un niveau abaissé de concentration du pepsinogène I est indicatif d'une atrophie fundique.

5. Procédé selon une ou plusieurs des revendications 1-2, dans lequel un taux accru de concentration de pepsinogène I est indicatif d'une gastrite fundique.

6. Procédé selon une ou plusieurs des revendications 1 - 2, dans lequel un niveau accru de concentration de pepsinogène I est indicatif d'une gastrite fundique, sans implication d'auto-immunité.

7. Procédé selon une ou plusieurs des revendications 1-2, dans lequel le niveau d'anticorps de la H,K-ATPase différant de celui de la population normale est indicatif d'une atrophie fundique auto-immune.

8. Procédé selon une ou plusieurs des revendications 1-2, dans lequel le niveau d'anticorps de Helicobacter pylori différant de celui de la population normale est indicatif d'une gastrite antrale ou d'une pangastrite.

9. Procédé selon une ou plusieurs des revendications 1-2, dans lequel les niveaux d'anticorps de Helicobacter pylori et les concentrations normales à réduites de pepsinogène I sont indicatifs d'une atrophie.

10. Procédé selon une ou plusieurs des revendications 1-2, dans lequel des concentrations très faibles de pepsinogène I associées à des niveaux accrus d'anticorps de la H,K-ATPase sont indicatifs d'une atrophie fundique.
